Europäisches Patentamt

European Patent Office

Office européen des brevets

(11) Publication number: **0 175 664**
**A2**

(19)

(12) # EUROPEAN PATENT APPLICATION

(21) Application number: **85850276.8**

(22) Date of filing: **04.09.85**

(51) Int. Cl.⁴: **A01N 41/04 , A61L 2/16**

(30) Priority: **12.09.84 SE 8404560**

(43) Date of publication of application:
**26.03.86 Bulletin 86/13**

(84) Designated Contracting States:
**AT BE CH DE FR GB IT LI LU NL SE**

(71) Applicant: **AB ÖRESTADS KEM.-TEKN. FABRIKER**
**Industrigatan 11**
**S-234 00 Lomma(SE)**

(72) Inventor: **Bladh, Einar**
**Box 503**
**S-240 21 Löddeköpinge(SE)**

(74) Representative: **Wiklund, Erik et al**
**AWAPATENT AB Box 5117**
**S-200 71 Malmö(SE)**

(54) **Decontaminating composition.**

(57) A decontaminating composition to be used, for example, for preserving medical preparations, in stomatal pouches, in latrines or animal sheds. The composition has the form of a powder or an aqueous solution and contains, as the active main constituent, a hydroxymethane sulphonic acid salt, preferably the sodium salt of hydroxymethane sulphonic acid. Preferably, the pulverulent composition comprises hydroxymethane sulphonic acid salt only, whereas the aqueous composition which preferably contains about 20-40 % by weight of an active main constituent, may also comprise optional additives, such as 0.001-1 % by weight of a surface tension depressor which preferably is a cetyl pyridinium chloride, and about 1-10 % by weight of iron or zinc salt for binding hydrogen sulphide. The preferred iron and zinc salts are iron sulphate and zinc chloride, respectively.

EP 0 175 664 A2

## DECONTAMINATING COMPOSITION

The present invention relates to a decontaminating composition in the form of a powder or an aqueous solution.

Under appropriate conditions, organic materials (also called substrates) are subjected to microbial degradation which is either aerobic (requiring the presence of oxygen) or anaerobic (requiring the absence of oxygen). Aerobic degradation occurs under admission of air and is, in actual practice, a surface process, whereas anaerobic degradation develops in the absence of air, for example under a liquid surface. The object of the microbial degradation process is to recover energy from the organic substrate which is composed of high-energy substances, such as carbohydrate, fat and protein. In the degradation process, this energy is recovered and stored in some suitable form, such as the high-energy substance ATP (adenosine triphosphate).

The aerobic process is characterised by a high yield of ATP per consumed quantity of substrate, i.e the degree of efficiency of the process is high. This makes the substrate last for a long time, and as a practical consequence, the degradation process is steady. The end products of the degradation process are the energy-poor substances carbon dioxide, water and nitrogen.

The anaerobic degradation process, however, is characterised by a low yield of ATP per consumed quantity of substrate, i.e. the degree of efficiency is low. As a practical consequence, large quantities of substrate are used. In the anaerobic degradation process, the energy which is not stored in the form of ATP, is present in residual products, inter alia some high-energy gaseous substances, such as hydrocarbons (substantially methane), mercaptans, hydrogen sulphide and ammonia. Non-gaseous, high-energy substances are, for example, alcohols produced in fermentation processes. In closed systems under controlled conditions, for example in sewage treatment plants, the energy in the gaseous products is utilised for the production of heat or power, simultaneously as the waste quantity is reduced considerably. To prevent the process from stopping for lack of some vital component, for example urea is added to supply nitrogen. Under uncontrolled conditions, for example in animal sheds, liquid-manure pits and latrines, the gaseous residuals are detrimental to the environment. Thus, methane constitutes an explosion hazard and may suffocate men and animals by displacement of air in poorly ventilated premises. Mercaptans, above all hydrogen sulphide, are extremely toxic, and a long period of exposure may result in chronic injuries, unpalatable food etc. Even very moderate concentrations can be lethal. Death may readily occur when ventilation is poor since the anaerobic degradation may become violent and liberate large quantities of hydrogen sulphide. Ammonia is not only highly toxic; it also has a corroding effect on metal fittings in animal sheds, caravans etc.

As is evident from the above, uncontrolled anaerobic degradation of an organic substrate implies great difficulties. Therefore it is important to take steps to protect men, animals and environment. These steps can be directed either to the micro-organism proper or to the gaseous products formed. For example, the micro-organism can be controlled by chemical means, such that it is destroyed (bactericidal-fungicidal effect), or such that its growth is inhibited (bacteriostatic-fungistatic effect). The effect obtained usually depends on the concentration of the chemical means. When the steps are directed to the gaseous products formed, the gases may be eliminated by ventilation, or the gases (except for methane) may be chemically bonded by means of a suitable preparation.

The present invention relates to a composition which can both inhibit or kill the micro-organisms and react with toxic gases formed.

To this end, the invention proposes the use of a salt of hydroxymethane sulphonic acid as the active main constituent in the composition. Preferably, the salt is an alkali metal salt, and the most preferred salt is the sodium salt of hydroxymethane sulphonic acid. This is an odorless and relatively non-poisonous salt.

The hydroxymethane sulphonic acid salt used in the composition has a strong microbial effect and, depending on its concentration in the composition, may have either a bactericidal or a bacteriostatic effect. In a system in which a certain anaerobic degradation process has already started, the hydroxymethane sulphonic acid salt can react with mercaptans, hydrogen sulphide and ammonia under formation of non-volatile, odorless substances. This double function of the odorless and relatively non-poisonous hydroxymethane sulphonic acid salt in the composition according to the invention is unique.

Further characteristic features of the invention are defined in the appended claims.

SE-patent 7107739-0 discloses the use of organic hydroxy sulphonate compounds, including hydroxymethane sulphonic acid salt, in plating solutions in order to improve the tolerance to the negative effects of metallic pollutants and to relatively high concentrations of primary brighteners. Said patent does not disclose the bactericidal/bacteriostatic effect of hydroxymethane sulphonic acid salts.

Furthermore, DE-AS 1,173,059 discloses the use of methylol sulphonic acid salt for the manufacture of creaseproof keratin-containing fiber materials, such as woolen fabrics. The fabric is impregnated with a solution of the methylol sulphonic acid salt and is treated under heat (60-110°C) and pressure for about 3-20 minutes.

Chemical Abstracts, Vol. 30 (1936), abstract No. 1737[3] discloses addition compounds of bisulfite and aldehydes, the addition compound of $H_2SO_3$ and HCHO being mentioned. This reference shows only that hydroxymethane sulphonic acid salts and the preparation thereof are per se known, but discloses in no way the bactericidal or bacteriostatic effect or the advantageous use thereof in decontaminating compositions. What has just been said is also relevant to Chemical Abstracts, Vol. 40 (1946), abstract No. 5012[7].

The Merck Index, Tenth Edition, 1983, p. 604, 4419 discloses hydroxymethane sulphonic acid sodium salt as such and states its chemical name, formula and use. It is to be noted that the only uses mentioned are those of fixing agents for keratin-containing fibers, the flotation of lead-zinc ores and the protection of color pictures. No bactericidal or bacteriostatic effect or use in decontaminating compositions is mentioned.

Finally, DE-OS 2,727,564 teaches a method for the preparation of mixtures of alkali-2-hydroxyalkyl sulphonates and alkylene glycols. The alkali-2-hydroxyalkyl sulphonates described contain 2 carbon atoms and are not equivalent to the hydroxymethane sulphonic acid salt according to the present invention.

As mentioned by way of introduction, the composition according to the invention can be either a powder or an aqueous solution, depending on the contemplated use. Furthermore, in addition to the active main constituent of hydroxymethane sulphonic acid salt, the composition may comprise one or more additives.

When the composition is pulverulent, like when used in stomatal pouches, it consists preferably of hydroxymethane sulphonic acid salt alone, i.e. further additives are usually not included. When the composition is in the form of an aqueous solution, it is preferably a concentrated solution comprising about 20-40 % by weight of hydroxymethane sulphonic acid salt. This concentrated solution may then be used either undiluted, for example for preserving medical preparations, or diluted to various desired concentrations, for example to about 1-5 % by weight, preferably about 2 % by weight of hydroxymethane sulphonic acid salt when used in latrines or animal sheds.

The optimal additives mentioned above are preferably included in the composition when this is in the form of an aqueous solution. The additives are, primarily, surface tension depressors and agents for further intensifying the hydrogen sulphide binding effect.

The surface tension depressor may be optionally chosen from known surface tension depressors, such as nonionic, anionic or cationic surface active agents, but is preferably chosen from agents which furthermore have a bacteriostatic or bactericidal effect, such as quaternary ammonia compounds. Such preferred surface tension depressors are, for example, benzalkonium chloride and cetyl pyridinium chloride (CPC), the latter being especially preferred. When the composition according to the invention comprises a surface tension depressor, the latter has a concentration of 0.001-1 % by weight, preferably about 0.3 % by weight.

An additive for binding hydrogen sulphide is an iron salt or a zinc salt, the preferred iron salt being iron sulphate, and the preferred zinc salt being zinc chloride.

Besides the above-mentioned additives, other known additives may also be included in the composition, for example a perfume which is added in a concentration enough to give the desired aroma.

Different applications of the composition according to the invention are further described below.

As already mentioned, the composition according to the invention can be used for preserving medical preparations (tissue samples, such as dissections from corpses) to be used for reference or education purposes. Since, on such applications, no microbial degradation whatever is allowed, the hydroxymethane sulphonic acid concentration of the composition must be kept high, and therefore the composition is in the form of a concentrated aqueous solution having a hydroxymethane sulphonic acid salt content of about 20-40 %. The practical upper limit is about 40 % by weight of hydroxymethane sulphonic acid salt since this corresponds approximately to a saturated solution, and higher concentrations merely cause the excess of hydroxymethane sulphonic acid salt to be precipitated in the solid form. Besides the active constituent of hydroxymethane sulphonic acid salt, no further additives are required when the composition is used for preserving medical preparations. The composition according to the invention can also be used in stomatal pouches, in which case it can be in solid form as a powder. The warm intestinal liquid dissolves the active hydroxymethane sulphonic acid salt so that a solution having a concentration of preferably about 5-10 % by weight of hydroxymethane sulphonic acid salt is obtained in the stomatal pouch. Preferably, the powder according to the invention added to the stomatal pouch comprises merely the active hydroxymethane sulphonic acid salt, and no further additives are required.

The composition according to the invention can also be used as sanitary liquid in latrines for caravans, boats, buses and airplanes. In this context, use is made of a concentrated solution (about 20-40 % by weight of hydroxymethane sulphonic acid salt) as mentioned above which is added to the empty receptacle and diluted with water to about 2 % by weight of hydroxymethane sulphonic acid salt to give bactericidal effect. As the receptacle is being filled, the bactericidal effect is weakened and turns bacteriostatic. When the latrine is finally emptied in the municipal sewer system, the degree of dilution is so high that there is no effect left. This is important because most sewage treatment plants comprise a biological step which is not able to withstand anti microbial agents. When being used in latrines, the composition according to the invention comprises also a surface tension depressor for dissolving lumps of faeces. It is preferred to use cetyl pyridinium chloride as surface tension depressor which also intensifies the antibacterial function. In order to intensify the hydrogen sulphide absorbing function, the composition preferably comprises also a zinc salt, such as zinc chloride. In this connection, iron salts are less preferred since they may cause staining of the latrine equipment.

A further application of the composition according to the invention is for binding gases in anaerobic degradation processes in connection with animals. Modern poultry-farms and animal sheds are provided with collecting ducts for the manure of the animals, in which ducts the collected liquid may either circulate, as in poultry-farms, or be stagnant, as in pig houses. After a certain period of time, the contents of the collecting ducts are discharged into large tanks and subsequently spread as fertiliser. The animals spend their entire life above their manure in the collecting ducts in which an intense anaerobic degradation occurs and large quantities of toxic hydrogen sulphide and ammonia are produced. If these gases are not extracted by means of efficient fans, it is possible, for example on power failure, that in a couple of hours lethal concentrations are formed which eliminate the entire stock of animals. As in latrine use, the composition according to the invention is added to the emptied system of collecting ducts in the form of a concentrated aqueous solution (about 20-40 % by weight of hydroxymethane sulphonic acid salt) which is diluted with a certain amount of water such that the initial concentration is about 2 % by weight of hydroxymethane sulphonic acid salt. Furthermore, the composition comprises a surface tension depressor (preferably CPC) and an additive, such as iron salt for binding hydrogen sulphide. As liquid manure is supplied to the collecting ducts and the concentration of the active constituent is reduced, further doses of the composition according to the invention should be added. The periodical adding of the composition according to the invention is adjusted to the volume of the collecting ducts and to the interval of emptying. After storing in tanks for a certain period of time, the manure is spread as fertiliser, and then the composition according to the invention, when in contact with soil, sun and air, is quickly degraded to harmless sodium sulphate.

Some formulations of the composition according to the invention are given below.

1. Composition for preserving dissections from corpses

A solution having 40 % by weight of sodium salt of hydroxymethane sulphonic acid in water.

2. Composition for use in stomatal pouches

100 % by weight of sodium salt of hydroxymethane sulphonic acid (powder).

3. Composition for use in latrines or animal sheds

40 % by weight of sodium salt of hydroxymethane sulphonic acid
0.3 % by weight of CPC
5 % by weight of zine chloride/iron sulphate
0.1 % by weight of perfume
water up to 100 % by weight.
The invention has been described above with reference to specific applications and compositions, but it will be appreciated that various modifications are possible within the scope of the invention as defined in the appended claims.

**Claims**

1. A decontaminating composition in the form of a powder or an aqueous solution, **characterised** in that it contains, in addition to optional additives, a hydroxymethane sulphonic acid salt as the active main constituent.

2. The composition as claimed in claim 1, **characterised** in that it contains an alkali metal salt of hydroxymethane sulphonic acid as the active main constituent.

3. The composition as claimed in claim 2, **characterised** in that it contains the sodium salt of hydroxymethane sulphonic acid as the active main constituent.

4. The composition as claimed in claim 1, in the form of an aqueous solution, **characterised** in that the concentration of the active main constituent is 20-40 % by weight.

5. The composition as claimed in claim 1, **characterised** in that it contains, as additives, a surface tension depressor and/or an iron or zinc salt.

6. The composition as claimed in claim 5, **characterised** in that is contains 0.001-1 % by weight of a surface tension depressor.

7. The composition as claimed in claim 6, **characterised** in that the surface tension depressor is cetyl pyridinium chloride.

8. The composition as claimed in claim 5, **characterised** in that it contains 1-10 % by weight of iron or zinc salt.

9. The composition as claimed in claim 8, **characterised** in that the iron salt is iron sulphate.

10. The composition as claimed in claim 8, **characterised** in that the zinc salt is zinc chloride.